# EUROPEAN PATENT APPLICATION

(11) **EP 3 819 373 A1**
(43) Date of publication of application: **12.05.2021**
(21) Application number: 19831274.6
(22) Date of filing: 04.07.2019
(51) Int. Cl.: C12N 5/077, C12M 3/02

(54) **CELL SHEET MANUFACTURING METHOD, CARDIAC MUSCLE CELL SHEET, AND KIT FOR MANUFACTURING CARDIAC MUSCLE CELL SHEET**

(30) Priority: 06.07.2018 JP 2018129380
(71) Applicant: Myoridge Co. Ltd., Kyoto-shi, Kyoto 606-8501 (JP)
(72) Inventor: MINAMI, Itsunari, Kyoto-shi, Kyoto 606-8501 (JP); SUETA, Shinichi, Kyoto-shi, Kyoto 606-8501 (JP); NAKAMURA, Toyomi, Kyoto-shi, Kyoto 606-8501 (JP); FUJIKAWA, Saori, Kyoto-shi, Kyoto 606-8501 (JP); MINATOHARA, Maiko, Kyoto-shi, Kyoto 606-8501 (JP)
(74) Representative: AWA Sweden AB
(86) International application number: PCT/JP2019/026660
(87) International publication number: WO 2020/009188

(57) **Abstract**

A method for producing a cell sheet, the method including subjecting animal cells dispersed in a culture medium to a suspension culture while gathering the animal cells toward the center of the culture medium, to form a cell sheet floating in the culture medium.

## Description

### FIELD

The present invention relates generally to a method for producing a cell sheet, a cardiomyocyte sheet, and a kit for producing a cardiomyocyte sheet.

### BACKGROUND

A cell sheet for transplantation is prepared by culturing cells with adhered on a substrate, such as a bottom surface of a petri dish, with the use of the adhesion force of the cells (see, for example, Patent Literature 1). The cell sheet formed on the support by adhesion culture needs to be peeled off from the support at the time of use. Due to the adhesion force of the cells, however, it is difficult to peel off the cell sheet from the support while maintaining the form of the cell sheet.

### CITATION LIST

### PATENT LITERATURE

Patent Literature 1: WO2011/058813

### SUMMARY

### TECHNICAL PROBLEM

It is an object of the present invention to provide a technique for producing a cell sheet without breakage of the cell sheet caused by peeling of the cell sheet from a support.

### SOLUTION TO PROBLEM

According to one aspect, there is provided a method for producing a cell sheet, the method comprising subjecting animal cells dispersed in a culture medium to a suspension culture while gathering the animal cells toward the center of the culture medium, to form a cell sheet floating in the culture medium.

According to another aspect, there is provided a cardiomyocyte sheet having a cardiomyocyte purity of 80 to 100%, preferably 85 to 100%, more preferably 90 to 100%, still more preferably 95 to 100%, and most preferably 98 to 100%.

According to further another aspect, there is provided a kit for producing a cardiomyocyte sheet, the kit comprising: iPS cell-derived cardiomyocytes; a culture medium for culturing the cardiomyocytes; and a suspension culture vessel for containing the culture medium.

### ADVANTAGEOUS EFFECTS OF INVENTION

According to the present invention, a technique for producing a cell sheet without breakage of the cell sheet caused by peeling of the cell sheet from a support can be provided.

### BRIEF DESCRIPTION OF THE DRAWINGS

FIG. 1 is a diagram for illustrating "gathering animal cells toward the center of a culture medium".
FIG. 2 is a schematic diagram showing a preferred embodiment of the method of the present invention.
FIG. 3A is a photograph showing an example of producing a cardiomyocyte sheet in a 24-well plate.
FIG. 3B is a photograph showing an example of producing a cardiomyocyte sheet in a 24-well plate.
FIG. 4 is a photograph showing an example of producing a cardiomyocyte sheet in a 6-cm dish.
FIG. 5 is a photograph showing an example of producing a cardiomyocyte sheet in a 10-cm dish.
FIG. 6 is a photograph showing an example of producing a cardiomyocyte sheet in a culture medium free from a thickening agent.
FIG. 7 is a photograph showing an example of producing a cardiomyocyte sheet having a cardiomyocyte purity of 98%.
FIG. 8 is a photograph showing an example of producing a mouse fibroblast sheet.
FIG. 9 is a photograph showing an example of producing a human iPS cell sheet.
FIG. 10 is a photograph showing an example of producing a cardiomyocyte sheet having a quadrangular shape.
FIG. 11 is a photograph showing an example of producing a human mesenchymal stem cell sheet.

### DETAILED DESCRIPTION

### 1. Method for producing cell sheet

A method for producing a cell sheet includes subjecting animal cells dispersed in a culture medium to a suspension culture while gathering the animal cells toward the center of the culture medium, to form a cell sheet floating in the culture medium.

### 1-1. Animal cells

The animal species of the animal cells used in this method is not particularly limited; however, it is preferably a mammal, a rodent, more preferably a primate, and still more preferably a human.

The animal cells are not particularly limited; examples thereof include differentiated cells derived from pluripotent stem cells, differentiated cells taken from an animal tissue, pluripotent stem cells, and somatic stem cells. "Pluripotent stem cell" refers to a cell having pluripotency, which is an ability to differentiate into any type of cell constituting a living body, and a self-replication capability, which is an ability to maintain the pluripotency even after cell division. The term "pluripotent stem cell" includes embryonic stem cells (ES cells), embryonic germ cells (EG cells), and induced pluripotent stem cells (iPS cells). "Somatic stem cell" refers to a cell having limited differentiation potency, which is an ability to differentiate into a specific type of cell, and a self-replication capability, which is an ability to maintain the limited differentiation potency even after cell division. The term "somatic stem cell" includes mesenchymal stem cells.

The animal cells are preferably differentiated cells derived from pluripotent stem cells, more preferably iPS cell-derived differentiated cells, still more preferably iPS cell-derived cardiomyocytes, and most preferably human iPS cell-derived cardiomyocytes. Alternatively, the animal cells are preferably somatic stem cells, more preferably mesenchymal stem cells, and still more preferably human mesenchymal stem cells.

When the animal cells are differentiated cells derived from pluripotent stem cells, the animal cells can be prepared from pluripotent stem cells by a known differentiation induction method. Alternatively, commercially available differentiated cells derived from pluripotent stem cells may be used. When the animal cells are somatic stem cells, somatic stem cells taken from an animal or commercially available somatic stem cells may be used.

When the animal cells are differentiated cells derived from pluripotent stem cells, the cells preferably have a high differentiated-cell purity, such as a differentiated-cell purity of 80 to 100%. When the animal cells are iPS cell-derived cardiomyocytes, the animal cells include the iPS cell-derived cardiomyocytes at a cardiomyocyte purity of preferably 80 to 100%, more preferably 85 to 100%, still more preferably 90 to 100%, still more preferably 95 to 100%, and most preferably 98 to 100%. The percentage of the cell purity as used in this specification represents a percentage based on the number of cells. The cardiomyocyte purity can be determined by a flow cytometry analysis using an antibody to a myocardial marker such as cardiac troponin T (cTnT), α-actinin, α-cardiac actin, or GATA-4.

The iPS cell-derived cardiomyocytes with a high cardiomyocyte purity can be prepared by, for example, a known method for inducing protein-free cardiac differentiation (PFCD) (see WO2015/182765). Since the method for inducing protein-free cardiac differentiation (PFCD) can achieve high myocardial differentiation efficiency, iPS cell-derived cardiomyocytes prepared by the method can achieve a high cardiomyocyte purity.

### 1-2. Culture medium and culture vessel

The culture medium to be used can be a known culture medium having a composition suitable for culturing animal cells that are used. When the animal cells are cardiomyocytes, a culture medium known as a culture medium for culturing cardiomyocytes can be used. Preferably, the culture medium for culturing cardiomyocytes contains inorganic salts including calcium salt, magnesium salt, potassium salt, and sodium salt, and a buffer solution. More preferably, a basal medium such as DMEM, RPMI, IMDM, or Ham-12 may be used as the culture medium for culturing cardiomyocytes. The basal medium is available from, for example, Sigma-Aldrich Japan. For example, a culture medium having the following composition may be used as the culture medium for culturing cardiomyocytes:
0.01 to 0.5 g/L (e.g., 0.182 g/L) of CaCl₂,
0 to 1.0 g/L (e.g., 0.09767 g/L) of MgSO₄,
0.1 to 1.0 g/L (e.g., 0.4 g/L) of KCl,
0 to 10.0 g/L (e.g., 3.362 g/L) of NaHCO₃,
1.0 to 20.0 g/L (e.g., 5.4525 g/L) of NaCl,
0 to 1.0 g/L (e.g., 0.109 g/L) of Na₂HPO₄, and
0 to 20.0 g/L (e.g., 5.958 g/L) of HEPES.

The culture medium may be free from a serum or contain a serum in an amount of 40% by mass or less with respect to the culture medium before addition of the serum. The pH of the culture medium is not particularly limited; however, it is preferably 6.0 to 9.0, and more preferably 7.0 to 8.0.

For a cell suspension culture and sheet formation, the culture medium is preferably put in a vessel such that the depth of the culture medium is, for example, 5 mm or more, preferably 5 to 50 mm.

From the viewpoint of the production efficiency of the cell sheet, the culture medium preferably further contains a thickening agent. When a thickening agent is contained in the culture medium, viscosity is imparted to the culture medium. As a result, the animal cells are less likely to sink to the bottom surface of the vessel in the culture medium, and can stably maintain the floating state. Also, the friction between the culture medium and the animal cells is reduced, so that the animal cells easily move in the culture medium. When a thickening agent is in the culture medium, the animal cells are easy to gather to form a sheet shape while floating in the culture medium, allowing for enhancement of the efficiency of sheet formation. On the other hand, from the viewpoint of utilizing the cell sheet for transplantation, the culture medium is preferably free from a thickening agent to eliminate the possibility of adhesion of the thickening agent to the cell sheet.

The thickening agent to be used is not particularly limited as long as it can impart viscosity to the culture medium, and examples thereof include methylcellulose, polyethylene glycol, xanthan gum, gellan gum, collagen, hyaluronic acid, and Hystem (registered trademark). The thickening agent can be added to the culture medium in an amount necessary for preparing a culture medium having a viscosity of, for example, 10 to 3000 mPa·S, preferably 50 to 1000 mPa·S. That is, the culture medium containing the thickening agent has a viscosity of, for example, 10 to 3000 mPa·S, preferably 50 to 1000 mPa·S. "Viscosity" as used in this specification refers to a viscosity at 25 °C. When methylcellulose (for example, HSC001 from R&D Systems, Inc.) is used as the thickening agent, it can be added to the culture medium at a concentration of, for example, 0.001 to 0.01 g/mL in terms of methylcellulose weight.

Any vessel suitable for a suspension culture can be used as the vessel for containing the culture medium. The vessel preferably has an inner wall with low cell adhesiveness. For example, a vessel with a coating for inhibiting adhesion of cells to the vessel applied to its inner wall can be used. Such a suspension culture vessel is well known and commercially available. For example, coating with Poly-HEMA, that is, poly(2-hydroxyethyl methacrylate), or with MPC, that is, 2-methacryloylethyl phosphorylcholine, is known as the coating for inhibiting the adhesion.

The vessel is preferably a flat-bottomed vessel. A flat-bottomed vessel is suitable for gathering animal cells during a suspension culture to form a sheet shape. The shape of the bottom surface of the flat-bottomed vessel is discretionary, and may be circular or quadrangular. In the method of the present invention, a cell sheet having a shape similar to that of the bottom surface of the flat-bottomed vessel can be produced. Therefore, when a circular cell sheet is to be produced, a flat-bottomed vessel having a circular bottom surface is preferably used.

The vessel to be used can be a commercially available flat-bottomed vessel for a suspension culture, such as a dish for a suspension culture, a flask for a suspension culture, or a plate for a suspension culture (i.e., a microtiter plate). More specifically, a dish for a suspension culture having a diameter of 3.5 to 10 cm, a flask for a suspension culture having a culture area of 12.5 to 225 cm², a 6-well plate for a suspension culture, a 12-well plate for a suspension culture, a 24-well plate for a suspension culture, a 96-well plate for a suspension culture, or the like can be used. For example, a vessel commercially available as a cell culture vessel having an ultra-low attachment surface, such as an ultra-low attachment dish (Corning Inc.), an ultra-low attachment flask (Corning Inc.), an ultra-low attachment plate (Corning Inc.), PrimeSurface dish (Sumitomo Bakelite Co., Ltd.), or the like can be used.

### 1-3. Suspension culture

The animal cells are seeded and dispersed in the culture medium in order to perform a suspension culture. At this time, the animal cells are preferably dispersed in the culture medium in a state of single cells, that is, in a state where the cells are discrete. The animal cells to be seeded in the culture medium can be prepared by treating a cell mass with a protease.

The animal cells are preferably present in the culture medium at a cell density suitable for cell sheet formation at the start of a suspension culture. The animal cells are preferably present at a cell density of, for example, 0.1 × 10⁶ to 10 × 10⁶ cells/cm², preferably 2 × 10⁶ to 4 × 10⁶ cells/cm² at the start of a suspension culture.

In the method of the present invention, the animal cells dispersed in the culture medium are subjected to a suspension culture while being gathered toward the center of the culture medium. Preferably, the animal cells dispersed in the culture medium are subjected to a suspension culture while being gathered toward the center of the culture medium so as to form a sheet shape as a whole.

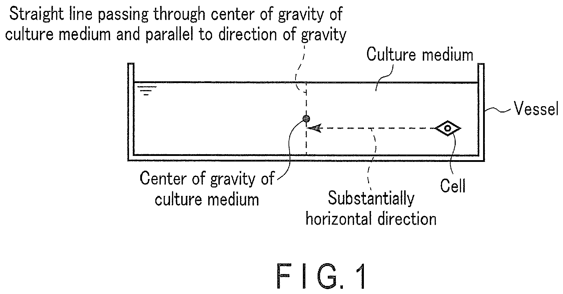

FIG. 1 shows a diagram for illustrating "gathering animal cells toward the center of a culture medium". The expression "gathering animal cells toward the center of a culture medium" as used in this specification refers to gathering animal cells so that each cell in the culture medium moves toward "a straight line passing through the center of gravity of the culture medium and parallel to the direction of gravity" (see FIG. 1). Preferably, the expression "gathering animal cells toward the center of a culture medium" refers to gathering animal cells so that each cell in the culture medium moves in a substantially horizontal direction toward "a straight line passing through the center of gravity of the culture medium and parallel to the direction of gravity" (see FIG. 1). The "substantially horizontal direction" as used in this specification refers to a direction at 90° ±10° with respect to the direction of gravity, preferably a direction at 90° ±5° with respect to the direction of gravity.

Gathering the animal cells toward the center of the culture medium increases the chance of contact between the cells during a suspension culture, whereby the cells adhere to each other, and repeating this allows for formation of a cell sheet floating in the culture medium without using a support.

Therefore, the suspension culture can be performed by any method as long as the animal cells can be gathered toward the center of the culture medium. The suspension culture can be performed preferably by applying vibration to the culture medium. Alternatively, the suspension culture may be performed by generating in the culture medium a sound wave directed toward the center of the culture medium.

The suspension culture can be performed under the culture conditions suitable for the animal cells that are to be used. In the case of cardiomyocytes, the suspension culture can be performed in an atmosphere of 5% CO₂ at a temperature of 20 to 43 °C. The suspension culture needs to be performed for a period necessary for forming a cell sheet, and is performed for, for example, six or more hours, preferably 12 to 24 hours.

In a preferred embodiment, the method for producing a cell sheet includes subjecting animal cells dispersed in a culture medium to a suspension culture while applying vibration to the culture medium, to form a cell sheet floating in the culture medium.

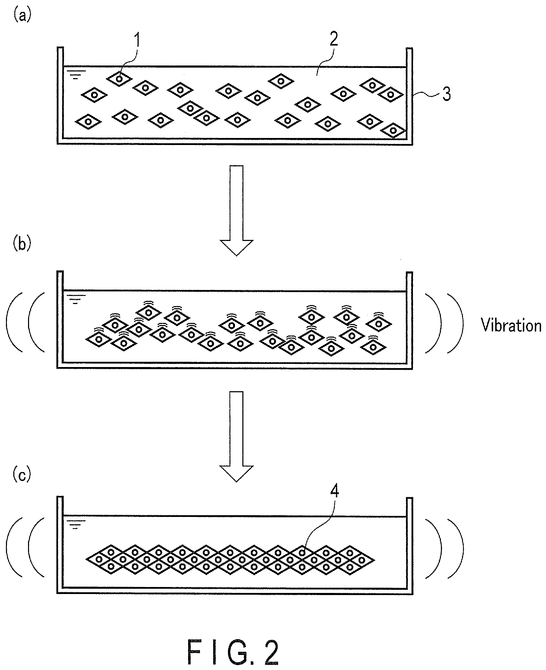

A preferred embodiment of the method of the present invention is schematically shown in FIG. 2. (a) in FIG. 2 shows a state in which animal cells 1 are dispersed in a culture medium 2 contained in a culture vessel 3; (b) in FIG. 2 shows a state in which the animal cells 1 gradually gather toward the center of the culture medium 2 when the animal cells 1 are subjected to a suspension culture while vibration is applied to the culture medium 2; and (c) in FIG. 2 shows a state in which as the suspension culture is continued, the animal cells 1 self-assemble into a sheet shape and adhere to each other to form a cell sheet 4.

Applying vibration to the culture medium increases the chance of contact between the cells during a suspension culture, whereby the cells adhere to each other, and repeating this allows for formation of a cell sheet floating in the culture medium without using a support.

Therefore, any vibration conditions can be adopted as long as the chance of contact between the cells during a suspension culture increases and the cells can thereby adhere to each other.

The vibration is preferably a vibration in a direction intersecting the direction of gravity, and more preferably a vibration in a direction substantially perpendicular to the direction of gravity. This enables the cells during a suspension culture to be efficiently brought into contact with each other and efficiently adhered to each other. "Direction substantially perpendicular to the direction of gravity" as used in this specification refers to a direction at 90° ±10° with respect to the direction of gravity, preferably a direction at 90° ±5° with respect to the direction of gravity. The vibration is more preferably a vibration in a direction perpendicular to the direction of gravity (i.e., a vibration in a horizontal direction).

The vibration may be a reciprocating vibration, but is preferably a rotational vibration. "Rotational vibration" as used in this specification refers to a vibration generated along with a rotational motion of a rotating body, and is, for example, a vibration in a circular motion.

The rotational vibration, for example, the vibration in a circular motion makes it possible to efficiently gather the cells toward the center of the culture medium. The vibration is still more preferably a vibration in a circular motion in a direction substantially perpendicular to the direction of gravity. The vibration is still more preferably a vibration in a circular motion in a direction perpendicular to the direction of gravity (i.e., a vibration in a circular motion in a horizontal direction).

The frequency of the vibration is generally 1 to 10000 Hz, preferably 2 to 3000 Hz, more preferably 5 to 100 Hz, and still more preferably 10 to 50 Hz, and the amplitude of the vibration is generally 0.1 to 10000 µm, preferably 0.5 to 100 µm, more preferably 0.5 to 10 µm, and still more preferably 1 to 10 µm. When the vibration is a rotational vibration, "amplitude" means an amplitude in a first direction perpendicular to the rotation axis and an amplitude in a second direction perpendicular to the rotation axis and the first direction.

The suspension culture with vibration applied can be performed using, for example, a vibration-applying apparatus. The vibration-applying apparatus includes, for example, an incubator body having a culture space for static culture of cells in its inside, and a vibration generator that is provided inside the incubator body and applies vibration to the culture vessel.

The incubator body is required to provide a sealed space controlled to satisfy environmental conditions (temperature, humidity, atmosphere, and the like) suitable for cell culture, and also required to allow the culture vessel to be taken in and out. Therefore, the incubator body has an opening and closing door on its front face. The incubator body usually includes a placement table for placing the culture vessel. The inner space of the incubator body may or may not be ventilated.

The vibration generator is provided inside the incubator body. The vibration generator may be arranged on the ceiling of the incubator body or on the placement table for placing the culture vessel. The vibration generator includes, for example, a motor. The vibration generator is preferably provided inside the incubator body so that the rotation axis of the motor is parallel to the direction of gravity.

The vibration is generally applied to the culture medium continuously over the entire period of the suspension culture. The vibration is preferably applied to the culture medium continuously at least until a cell sheet is formed. The vibration may be applied continuously or intermittently as long as cells can be gathered toward the center of the culture medium.

The completion of the production of a cell sheet can be determined by confirming the production of the cell sheet with the naked eye and confirming that the cell sheet maintains its integrity by shaking the culture vessel.

### 1-4. Effects

In the method of the present invention, a cell sheet floating in a culture medium can be formed by a suspension culture. Therefore, unlike the case where a cell sheet is produced on a support by adhesion culture, the method of the present invention does not require peeling of the cell sheet from a support, allowing the cell sheet to be used as is as a transplantation material. In addition, the method of the present invention can produce a cell sheet without breakage of the cell sheet caused by peeling of the cell sheet from a support.

The method of the present invention, which can produce a cell sheet by performing a suspension culture for about one day, is a very simple method that enables production of a cell sheet in a short period of time, and is suitable for mass production of cell sheets.

The method of the present invention can produce a cell sheet with a high purity of target cells to be formed into a sheet. That is, a cell sheet with a high cell purity can be produced without the need for additional cells such as fibroblasts, which have been needed in the production of conventional cell sheets. The inventors of the present invention have demonstrated production of a cardiomyocyte sheet with a cardiomyocyte purity of up to 98%. Since cardiomyocytes do not become cancerous, a cardiomyocyte sheet with a high cardiomyocyte purity is excellent in terms of the low risk of canceration after transplantation.

The method of the present invention can produce a cell sheet having a uniform shape. "Uniform shape" as used in this specification means that the cell sheet has a substantially uniform thickness over an area of 90% or more of the plane of the cell sheet when viewed with the naked eye and has a shape similar to that of the bottom surface of the culture vessel. The expression "the cell sheet has a substantially uniform thickness over an area of 90% or more of the plane of the cell sheet when viewed with the naked eye" means that a portion with an extremely small thickness may be present in a very small number of parts of the cell sheet (e.g., a very small number of parts at the edge or in the center of the sheet). In other words, as long as the cell sheet has a substantially uniform thickness over an area of 90% or more of the plane of the cell sheet when viewed with the naked eye, there is no practical problem. "Substantially uniform thickness" means, for example, that the minimum thickness and the maximum thickness fall within a range of ±25% of the intermediate value thereof, preferably within a range of ±20% of the intermediate value thereof, over an area of 90% or more of the plane of the cell sheet. Since the cell sheet is formed of an aggregate of cells dispersed in the culture medium and the shape of the cell sheet is difficult to control, it is clear that the expression "a shape similar to that of the bottom surface of the culture vessel" does not strictly mean a similar shape. The expression "uniform shape" is used in a sense that excludes a case where the cell sheet is broken and a case where the cell sheet is shrunk and has a curled shape. When a flat-bottomed vessel having a perfect circular bottom surface is used as the culture vessel, "uniform shape" means that the cell sheet has a substantially uniform thickness over the entire sheet and has a shape similar to a perfect circle (i.e., an approximately circular shape), when viewed with the naked eye.

In the method of the present invention, the size of a cell sheet can be freely controlled by changing the size of the bottom surface of the culture vessel, and a cell sheet having a relatively large size can be produced. For example, in the case of a circular sheet, a cell sheet having a diameter of 5 cm or more can be produced. Also, since the method of the present invention can produce a cell sheet having a shape similar to that of the bottom surface of the culture vessel, the shape of the cell sheet can be freely controlled by changing the shape of the bottom surface of the culture vessel.

Since the method of the present invention can produce a cell sheet having sufficient strength and that is easy to handle, there is an advantage wherein the cell sheet is easy to handle at the time of transplantation. For example, the cell sheet of the present invention can be scooped up with a spatula or the like and attached to a transplantation site.

On the other hand, in the conventional method (WO2011/058813), myoblasts and cells other than myoblasts (such as mesenchymal stem cells and fibroblasts) are mixed together and subjected to adhesion culture to produce a cell sheet, and it is essential to add cells other than the target cells to be formed into a sheet when forming a cell sheet. The conventional method (i.e., a method by adhesion culture using additional cells) is mainstream in this technical field as of the filing date of the present application, and clinical studies have just been started using cardiomyocyte sheets produced by the conventional method. The inventors of the present invention tried producing cardiomyocyte sheets having a cardiomyocyte purity of 80% or more by the conventional method (WO2011/058813); however, when the cell sheets were peeled off from a support, the cell sheets were either broken or shrunk and curled, making it impossible to produce sheets having a uniform shape.

As described above, according to the method of the present invention, a cell sheet having a uniform shape and sufficient strength was successfully produced even though there were almost no cells other than the target cells to be formed into a sheet. This is considered to be because the cells self-assembled into a sheet shape in a closely packed state with almost no gap therebetween and adhered to each other to form a sheet during the suspension culture. The method of the present invention, which overturns the common technical knowledge as of the filing of the present application that additional cells are essential for the production of a cell sheet and adopts an approach completely different from the conventional one to enable production of a cell sheet having a high cell purity, is an epoch-making method.

### 2. Cell sheet

According to another aspect, a cell sheet produced by the above-described method is provided. The cell sheet of the present invention has the effects described above in the section of "1-4. Effects".

Since the cell sheet of the present invention does not require additional cells, such as fibroblasts, other than the target cells to be formed into a sheet at the time of the production of the cell sheet, the purity of the target cells to be formed into a sheet is high. Specifically, the cell sheet of the present invention has a target-cell purity of preferably 80 to 100%, more preferably 85 to 100%, still more preferably 90 to 100%, still more preferably 95 to 100%, and most preferably 98 to 100%. The target-cell purity of the cell sheet is almost the same as the target-cell purity of the raw material cells used for sheet formation.

The cell sheet of the present invention preferably has a uniform shape. The cell sheet of the present invention is preferably free from cells other than the target cells. That is, the cell sheet of the present invention preferably has a target-cell purity of 100%. The cell sheet of the present invention is preferably free from fibroblasts.

The cell sheet of the present invention can have various sizes depending on the purpose. For example, the cell sheet can have a relatively small size when produced for transplantation into a small animal or for screening of drug response. Alternatively, the cell sheet can have a relatively large size when produced for transplantation into a large animal or for examination of drug response specific to a large tissue. The cell sheet of the present invention has a maximum length of, for example, 0.1 to 30 cm, preferably 1 to 10 cm. The "maximum length" as used in this specification refers to the length of the longest line segment among the line segments connecting two points on the contour of the plane of the cell sheet. When the cell sheet is circular, it has a maximum length of, for example, 0.1 to 30 cm, preferably 1 to 10 cm. When the cell sheet has a substantially perfect circular shape, it has a diameter of, for example, 0.1 to 30 cm, preferably 1 to 10 cm. When the cell sheet is quadrangular, it has a maximum length of, for example, 0.1 to 30 cm, preferably 1 to 10 cm. The cell sheet has a thickness of, for example, 0.01 to 1 cm.

The cell sheet may be a sheet of any cell type of any animal species, but is preferably a cardiomyocyte sheet, more preferably a human-cardiomyocyte sheet. The cardiomyocyte sheet of the present invention has the effects described above in the section of "1-4. Effects".

The cardiomyocyte sheet of the present invention has a high cardiomyocyte purity. Specifically, the cardiomyocyte sheet has a cardiomyocyte purity of preferably 80 to 100%, more preferably 85 to 100%, still more preferably 90 to 100%, still more preferably 95 to 100%, and most preferably 98 to 100%. The human-cardiomyocyte sheet has a human-cardiomyocyte purity of preferably 80 to 100%, more preferably 85 to 100%, still more preferably 90 to 100%, still more preferably 95 to 100%, and most preferably 98 to 100%. The cardiomyocyte purity of the cardiomyocyte sheet is almost the same as the cardiomyocyte purity of the raw material cells used for sheet formation.

The cardiomyocyte sheet of the present invention preferably has a uniform shape. The cardiomyocyte sheet of the present invention is preferably free from cells other than cardiomyocytes. That is, the cardiomyocyte sheet of the present invention preferably has a cardiomyocyte purity of 100%. The cardiomyocyte sheet of the present invention is preferably free from fibroblasts.

In addition, the cardiomyocyte sheet of the present invention can have various sizes depending on the purpose, and can have a relatively large size. The cardiomyocyte sheet of the present invention has a maximum length of, for example, 0.1 to 30 cm, preferably 1 to 10 cm. When the cardiomyocyte sheet is circular, it has a maximum length of, for example, 0.1 to 30 cm, preferably 1 to 10 cm. When the cardiomyocyte sheet has a substantially perfect circular shape, it has a diameter of, for example, 0.1 to 30 cm, preferably 1 to 10 cm. When the cardiomyocyte sheet is quadrangular, it has a maximum length of, for example, 0.1 to 30 cm, preferably 1 to 10 cm.

### 3. Kit for producing cardiomyocyte sheet

According to another aspect, there is provided a kit for producing a cardiomyocyte sheet, the kit including:
iPS cell-derived cardiomyocytes;
a culture medium for culturing the cardiomyocytes;
a suspension culture vessel for containing the culture medium.

In the kit of the present invention, the iPS cell-derived cardiomyocytes have a cardiomyocyte purity of preferably 80 to 100%, more preferably 85 to 100%, still more preferably 90 to 100%, still more preferably 95 to 100%, and most preferably 98 to 100%. As described above, the cardiomyocytes can be prepared by a known method for inducing protein-free cardiac differentiation (PFCD) (see WO2015/182765) .

The cardiomyocytes may be a cardiomyocyte mass prepared by a known method for inducing protein-free cardiac differentiation (PFCD), or a population of single cells obtained by treating a cardiomyocyte mass prepared by a known method for inducing protein-free cardiac differentiation (PFCD) with a protease. The "population of single cells" refers to cells that are not adhered to each other and are in a discrete state.

In the kit of the present invention, a culture medium known as a culture medium for culturing cardiomyocytes can be used as the culture medium. For example, the "culture medium for culturing cardiomyocytes" described above in the section of "1-2. Culture medium and culture vessel" can be used. As described above, the culture medium may be free from a serum, but preferably contains a serum. When the culture medium contains a serum, it can contain the serum in an amount of, for example, 40% by mass or less with respect to the culture medium before addition of the serum.

As described above in the section of "1-2. Culture medium and culture vessel", the culture medium may contain a thickening agent when the cell sheet is produced. Therefore, the culture medium included in the kit may contain a thickening agent in advance. In this case, the thickening agent is generally contained in the culture medium in an amount necessary for imparting a desired viscosity to the culture medium. Alternatively, the culture medium included in the kit may be free from a thickening agent, and a thickening agent may be included in the kit in a separate package from that of the culture medium. In this case, the thickening agent is generally included in the kit in an amount necessary for imparting a desired viscosity to the culture medium. When a thickening agent is included in the kit in a separate package from that of the culture medium, the user needs to add the thickening agent to the culture medium before producing the cell sheet.

In the kit of the present invention, the suspension culture vessel can be the culture vessel described above in the section of "1-2. Culture medium and culture vessel". That is, the exemplary culture vessels described above in the section of "1-2. Culture medium and culture vessel" can be used as the suspension culture vessel.

As described above, the suspension culture vessel preferably has an inner wall with a low cell adhesiveness. As described above, the suspension culture vessel preferably has a coating for inhibiting cell adhesion applied to its inner wall. For example, coating with Poly-HEMA, that is, poly(2-hydroxyethyl methacrylate), or with MPC, that is, 2-methacryloylethyl phosphorylcholine, is known as the coating for inhibiting the adhesion.

The kit of the present invention may further include instructions describing a procedure for producing a cardiomyocyte sheet. The instructions describe how to carry out the suspension culture described above in the section of "1-3. Suspension culture" to enable the user to produce a cardiomyocyte sheet.

The kit of the present invention includes the iPS cell-derived cardiomyocytes in an appropriate amount according to the size of the cell sheet to be produced, in other words, the size of the bottom surface of the suspension culture vessel. Also, the kit of the present invention includes the culture medium in an appropriate amount according to the size of the suspension culture vessel. For example, when a suspension culture vessel having a circular bottom surface with a diameter of 6 cm and a 10-mL capacity is included in the kit, 1 to 100 × 10e6 cardiomyocytes and a 1 to 1000 mL culture medium can be included in the kit.

### 4. Preferred Embodiments

The preferred embodiments of the present invention are summarized below.
[1] A method for producing a cell sheet, the method comprising subjecting animal cells dispersed in a culture medium to a suspension culture while gathering the animal cells toward the center of the culture medium, to form a cell sheet floating in the culture medium.
[2] The method according to [1], wherein the suspension culture is performed by applying vibration to the culture medium.
[3] A method for producing a cell sheet, the method comprising subjecting animal cells dispersed in a culture medium to a suspension culture while applying vibration to the culture medium, to form a cell sheet floating in the culture medium.
[4] The method according to [2] or [3], wherein the vibration is a vibration in a direction substantially perpendicular to a direction of gravity, preferably a vibration in a direction perpendicular to a direction of gravity.
[5] The method according to any one of [2] to [4], wherein the vibration is a rotational vibration.
[6] The method according to any one of [2] to [5], wherein the vibration is a vibration in a circular motion.
[7] The method according to any one of [2] to [6], wherein the vibration is a vibration in a circular motion in a direction substantially perpendicular to a direction of gravity, preferably a vibration in a circular motion in a direction perpendicular to a direction of gravity.
[8] The method according to any one of [2] to [7], wherein the vibration has a frequency of 1 to 10000 Hz, preferably 2 to 3000 Hz, more preferably 5 to 100 Hz, and still more preferably 10 to 50 Hz.
[9] The method according to any one of [2] to [8], wherein the vibration has an amplitude of 0.1 to 10000 µm, preferably 0.5 to 100 µm, more preferably 0.5 to 10 µm, and still more preferably 1 to 10 µm.
[10] The method according to any one of [1] to [9], wherein the culture medium has a depth of 5 mm or more, preferably 5 to 50 mm.
[11] The method according to any one of [1] to [10], wherein the culture medium comprises a thickening agent.
[12] The method according to any one of [1] to [11], wherein the thickening agent is contained in the culture medium in an amount necessary for preparing a culture medium having a viscosity of 10 to 3000 mPa·S, preferably 50 to 1000 mPa·S.
[13] The method according to any one of [1] to [12], wherein a vessel for containing the culture medium is a flat-bottomed vessel.
[14] The method according to any one of [1] to [13], wherein a vessel for containing the culture medium has a coating for inhibiting cell adhesion applied to an inner wall of the vessel.
[15] The method according to any one of [1] to [14], wherein the animal cells are present at a cell density of 0.1 × 10⁶ to 10 × 10⁶ cells/cm², preferably a cell density of 2 × 10⁶ to 4 × 10⁶ cells/cm², at a start of the suspension culture.
[16] The method according to any one of [1] to [15], wherein the suspension culture is performed in an atmosphere of 5% CO₂.
[17] The method according to any one of [1] to [16], wherein the suspension culture is performed at a temperature of 20 to 43 °C.
[18] The method according to any one of [1] to [17], wherein the suspension culture is performed for six or more hours, preferably 12 to 24 hours.
[19] The method according to any one of [1] to [18], wherein the animal cells are mammal or rodent cells, preferably primate cells, and more preferably human cells.
[20] The method according to any one of [1] to [19], wherein the animal cells comprise differentiated cells derived from pluripotent stem cells, differentiated cells taken from an animal tissue, pluripotent stem cells, or somatic stem cells.
[21] The method according to any one of [1] to [20], wherein the animal cells comprise differentiated cells derived from pluripotent stem cells.
[22] The method according to any one of [1] to [21], wherein the animal cells comprise iPS cell-derived differentiated cells.
[23] The method according to any one of [1] to [22], wherein the animal cells comprise iPS cell-derived cardiomyocytes.
[24] The method according to any one of [1] to [23], wherein the animal cells comprise iPS cell-derived cardiomyocytes at a cardiomyocyte purity of 80 to 100%, preferably 85 to 100%, more preferably 90 to 100%, still more preferably 95 to 100%, and most preferably 98 to 100%.
[25] The method according to [23] or [24], wherein the iPS cell-derived cardiomyocytes is human iPS cell-derived cardiomyocytes.
[26] The method according to any one of [1] to [20], wherein the animal cells are mesenchymal stem cells.
[27] The method according to any one of [1] to [21], wherein the animal cells are human mesenchymal stem cells.
[28] A cell sheet obtainable by the method according to any one of [1] to [27].
[29] The cell sheet according to [28], wherein the cell sheet has a cardiomyocyte purity of 80 to 100%, preferably 85 to 100%, more preferably 90 to 100%, still more preferably 95 to 100%, and most preferably 98 to 100%.
[30] A cardiomyocyte sheet obtainable by the method according to any one of [1] to [27].
[31] The cardiomyocyte sheet according to [30], wherein the cardiomyocyte sheet has a cardiomyocyte purity of 80 to 100%, preferably 85 to 100%, more preferably 90 to 100%, still more preferably 95 to 100%, and most preferably 98 to 100%.
[32] A cardiomyocyte sheet having a cardiomyocyte purity of 80 to 100%, preferably 85 to 100%, more preferably 90 to 100%, still more preferably 95 to 100%, and most preferably 98 to 100%.
[33] The cardiomyocyte sheet according to any one of [30] to [32], wherein the cardiomyocyte sheet has a uniform shape.
[34] The cardiomyocyte sheet according to any one of [30] to [33], wherein the cardiomyocyte sheet is free from a cell other than a cardiomyocyte.
[35] The cardiomyocyte sheet according to any one of [30] to [34], wherein the cardiomyocyte sheet has a maximum length of 0.1 to 30 cm, preferably 1 to 10 cm.
[36] The cardiomyocyte sheet according to any one of [30] to [35], wherein the cardiomyocyte sheet has a circular or quadrangular shape.
[37] The cardiomyocyte sheet according to any one of [30] to [36], wherein the cardiomyocyte sheet is a mammal or rodent cardiomyocyte sheet, preferably a primate cardiomyocyte sheet, and more preferably human cardiomyocyte sheet.
[38] A kit for producing a cardiomyocyte sheet, the kit comprising:
   iPS cell-derived cardiomyocytes;
   a culture medium for culturing the cardiomyocytes; and
   a suspension culture vessel for containing the culture medium.
[39] The kit according to [38], wherein the vessel has a coating for inhibiting cell adhesion applied to an inner wall of the vessel.
[40] The kit according to [38] or [39], wherein the cardiomyocytes have a cardiomyocyte purity of 80 to 100%, preferably 85 to 100%, more preferably 90 to 100%, still more preferably 95 to 100%, and most preferably 98 to 100%.
[41] The kit according to any one of [38] to [40], wherein the kit further comprises a thickening agent for imparting a viscosity to the culture medium.
[42] The kit according to any one of [38] to [41], wherein the vessel is a flat-bottomed vessel.
[43] The kit according to any one of [38] to [42], wherein the cardiomyocytes are mammal or rodent cardiomyocytes, preferably primate cardiomyocytes, and more preferably human cardiomyocytes.

### [EXAMPLES]

### [Example 1] Production of cardiomyocyte sheets

### (1) Method

A cardiomyocyte mass (i.e., an aggregate of cardiomyocytes having a size of several hundred microns to several millimeters) with a cardiomyocyte purity of 85 to 98% was prepared from human iPS cells (Center for iPS Cell Research and Application, Kyoto University, 253G1 strain) by a method for inducing protein-free cardiac differentiation (PFCD) (see WO2015/182765). The cardiomyocyte purity was determined by a flow cytometry analysis using an antibody to cTnT, which is a myocardial marker.

The obtained cardiomyocyte mass was treated with a protease solution containing 0.25% trypsin for 20 to 40 minutes to be dispersed into discrete single cardiomyocytes.

The dispersed single cardiomyocytes were centrifuged and the supernatant including the protease solution was discarded. Thereafter, the obtained cardiomyocytes were suspended in a DMEM or IMDM basal medium supplemented with 20% methylcellulose, 20% FBS, and 10 µM Y27632 (Rho-associated kinase inhibitor).

The suspended single cardiomyocytes were seeded in each of the culture vessels listed below at a cell density of 1 × 10⁶ to 4 × 10⁶ cells/cm², and the cells were agitated uniformly in the culture medium by pipetting. This allowed the cardiomyocytes to be dispersed in the culture medium in the form of single cells.

Ultra-low attachment 96-well plate (Corning Inc.)

Ultra-low attachment 24-well plate (Corning Inc.)

Ultra-low attachment 6-well plate (Corning Inc.)

Ultra-low attachment 6-cm dish (Corning Inc.)

Ultra-low attachment 10-cm dish (Corning Inc.)

The culture vessels were transferred to a vibration-applying apparatus equipped with an incubator body and a vibration generator, and the cells were cultured at 37 °C in a 5% CO₂ environment. The incubator body included a metal plate for placing the culture vessels. The vibration generator included a motor, and was arranged on the ceiling of the incubator body so that the rotation axis of the motor was parallel to the direction of gravity. The culture was performed while applying vibration to the culture medium. During the culture, the inner space of the incubator body was ventilated.

The vibration conditions were as follows (see the section of "(3) Measurement of vibration" below):
Type: vibration in a circular motion in a substantially horizontal direction
Frequency: 20 Hz (1200 rpm)
Amplitude: 0.5 to 3.0 µm

When implementing the method of the present invention, the culture vessel was let to stand directly on the metal plate and nothing was placed under the culture vessel. On the other hand, when implementing a method of a comparative example, an empty container identical to the culture vessel was placed under the culture vessel, or a vibration-proof material (a cushion) was placed under the culture vessel.

Whether or not cardiomyocyte sheets were formed was visually confirmed 20 to 24 hours after the culture was started.

### (2) Results

When the culture vessels were let to stand directly on the metal plate, circular cardiomyocyte sheets having a diameter of 0.1 to 8 cm were formed. Then, it was confirmed that the shape of the sheets was maintained in the circular shape even when the culture vessels were shaken by hand. Concrete results are as described below.

In the case of the ultra-low attachment 96-well plate, circular cardiomyocyte sheets having a diameter of about 1 mm to several millimeters were formed.

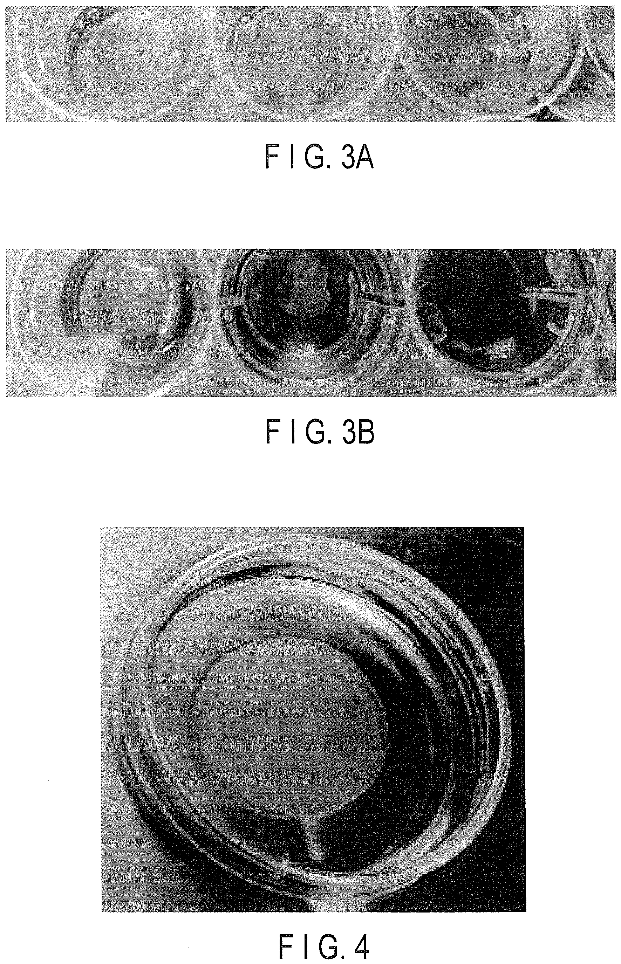

In the case of the ultra-low attachment 24-well plate, circular cardiomyocyte sheets having a diameter of about 0.5 to 1 cm were formed. Photographs of the obtained cardiomyocyte sheets are shown in FIGS. 3A and 3B.

In the case of the ultra-low attachment 6-well plate, circular cardiomyocyte sheets having a diameter of about 1 to 3 cm were formed.

In the case of the ultra-low attachment 6-cm dish, circular cardiomyocyte sheets having a diameter of about 2 to 5 cm were formed. A photograph of the obtained cardiomyocyte sheet is shown in FIG. 4.

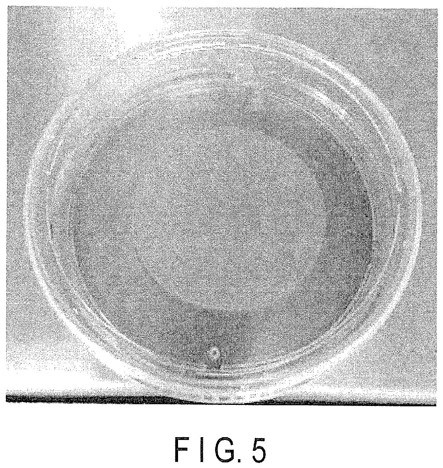

In the case of the ultra-low attachment 10-cm dish, circular cardiomyocyte sheets having a diameter of about 4 to 8 cm were formed. A photograph of the obtained cardiomyocyte sheet is shown in FIG. 5.

In all of the cardiomyocyte sheets, the pulsation and intracellular calcium fluorescence were confirmed.

All of the cardiomyocyte sheets had a cardiomyocyte purity of 85 to 98%, which was the same as the cardiomyocyte purity of the raw material cells used for sheet formation. Since the method of the present invention does not require additional cells other than the target cells in order to form a cell sheet, a cell sheet having a high cardiomyocyte purity was successfully produced using the raw material cells having a high cardiomyocyte purity.

All of the cardiomyocyte sheets had a uniform shape. That is, all of the cardiomyocyte sheets had a substantially uniform thickness over the entirety of the sheets and had an approximately circular shape, when observed with the naked eye.

All of the cardiomyocyte sheets had a sufficient strength and were able to be handled by scooping with a spatula.

The above results demonstrate that the method of the present invention can freely control the size of the cell sheet by changing the size of the bottom surface of the culture vessel.

On the other hand, when an empty container identical to the culture vessel was placed under the culture vessel, a plurality of discrete sheet pieces with holes were formed, making it impossible to form a single sheet with integrity. Also when a vibration-proof material (a cushion) was placed under the culture vessel, a plurality of discrete sheet pieces with holes were formed, making it impossible to form a single sheet with integrity.

### (3) Measurement of vibration

The vibration was measured by a vibration measuring apparatus (LK-G5000 Displacement Meter by KEYENCE CORPORATION). The vibration was measured at three positions where the metal plate (i.e., the table for placing the culture vessels), the culture vessels, and the main body of the vibration-applying apparatus were located.

The vibration of the metal plate in a horizontal direction was measured in the depth direction of the apparatus. The vibration of the metal plate in a horizontal direction had a frequency of 20 Hz (1200 rpm) and an amplitude of 0.5 to 3.0 µm.

The vibration of the culture vessels in a horizontal direction was measured in the depth direction of the apparatus. The vibration of the culture vessels in a horizontal direction had a frequency of 20 Hz (1200 rpm) and an amplitude of 0.5 to 3.0 µm.

The vibration of the vibration-applying apparatus in a horizontal direction was measured in both the width and depth directions of the apparatus. The vibration in the width direction had a frequency of 20 Hz (1200 rpm) and an amplitude of 0.5 to 3.0 µm. The vibration in the depth direction had a frequency of 20 Hz (1200 rpm) and an amplitude of 0.5 to 3.0 µm.

The same frequency and the same amplitude were measured at the three positions where the metal plate, the culture vessels, and the main body of the vibration-applying apparatus were located. The same frequency and the same amplitude were also measured in the width direction and the depth direction. From this, it is considered that the vibration-applying apparatus generated a vibration in a circular motion in a substantially horizontal direction.

On the other hand, when an empty container identical to the culture vessel was placed under the culture vessel, or a vibration-proof material (a cushion) was placed under the culture vessel, only a very small amplitude was occasionally observed when the vibration of the culture vessel in a horizontal direction was measured.

### (4) Discussion

The mechanism by which the cell sheets were formed in the Examples is assumed to be as follows. Due to the vibration in a circular motion in a substantially horizontal direction, strong vibration was applied to a portion of the culture medium near the side wall of the culture vessels, and weaker vibration was applied to a portion of the culture medium near the center of the culture vessels, as compared to the portion of the culture medium near the side wall of the culture vessels. Therefore, the cells dispersed in the culture medium gradually moved from a place where the cells were present to a place where weaker vibration was applied (i.e., toward the center of the culture medium), while vibrating, and gathered toward the center of the culture medium. It is considered that this resulted in formation of the cell sheets with their integrity maintained (see FIG. 2).

### [Example 2] Production of cardiomyocyte sheets using culture medium free from thickening agent

Cardiomyocyte sheets were produced in the same manner as in Example 1, except that 20% methylcellulose was not added to the culture medium. The ultra-low attachment 96-well plate (Corning Inc.) was used as the culture vessel. Cardiomyocytes were seeded in the culture vessel at a cell density of 1 × 10⁶ cells/cm² or 2 × 10⁶ cells/cm².

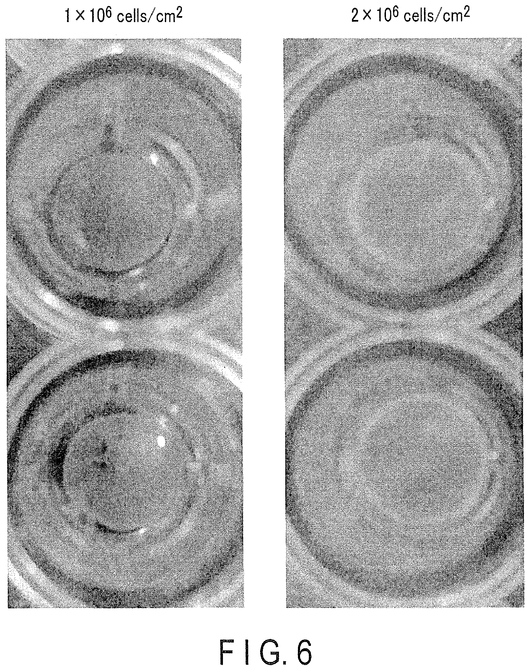

Circular cardiomyocyte sheets having a diameter of about 5 mm were successfully formed even without adding a thickening agent to the culture medium. Photographs of the obtained cardiomyocyte sheets are shown in FIG. 6. In FIG. 6, the photograph on the left side shows the case of a cell density of 1 × 10⁶ cells/cm², and the photograph on the right side shows the case of a cell density of 2 × 10⁶ cells/cm².

All of the cardiomyocyte sheets had a cardiomyocyte purity of 95.0%. All of the cardiomyocyte sheets had a uniform shape. That is, all of the cardiomyocyte sheets had a substantially uniform thickness over the entirety of the sheets and had an approximately circular shape, when observed with the naked eye. In addition, all of the cardiomyocyte sheets had sufficient strength and were able to be handled by scooping with a spatula.

### [Example 3] Production of cardiomyocyte sheet with 98% cardiomyocyte purity

A cardiomyocyte sheet was produced in the same manner as in Example 1, except that a mass of cardiomyocytes having a cardiomyocyte purity of 98% was prepared by differentiation induction and the cardiomyocytes were seeded in a culture vessel. The ultra-low attachment 6-cm dish (Corning Inc.) was used as the culture vessel. Cardiomyocytes were seeded in the culture vessel at a cell density of 2.2 × 10⁶ cells/cm².

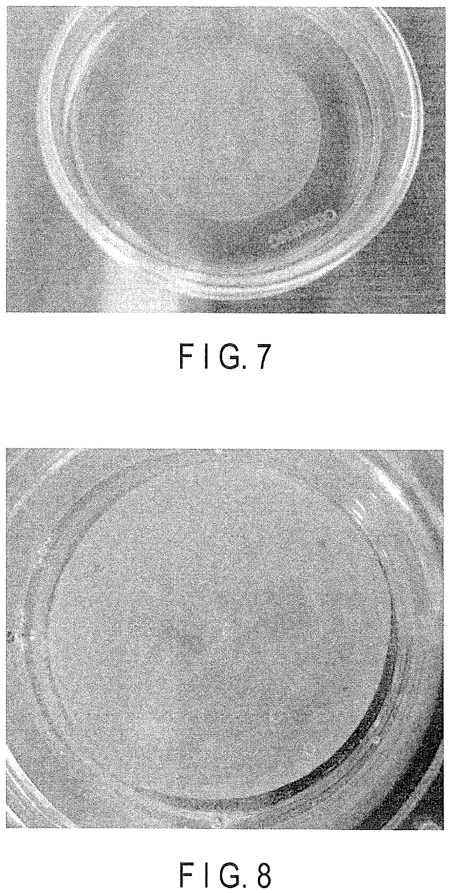

A cardiomyocyte sheet having a cardiomyocyte purity of 98% was successfully formed. The diameter was about 3 cm. A photograph of the obtained cardiomyocyte sheet is shown in FIG. 7.

The cardiomyocyte sheet had a uniform shape. That is, the cardiomyocyte sheet had a substantially uniform thickness over the entire sheet and had an approximately circular shape, when observed with the naked eye. In addition, the cardiomyocyte sheet had sufficient strength and was able to be handled by scooping with a spatula. A cardiomyocyte sheet having a high cardiomyocyte purity is excellent in that it is easy to control the quality thereof owing to the low proliferation capability of cardiomyocytes, and that the risk of tumorigenicity is low when the cardiomyocyte sheet is used for transplantation.

### [Example 4] Production of mouse fibroblast sheet and human iPS cell sheet

A cell sheet was produced in the same manner as in Example 1, except that mouse embryonic fibroblasts (MEF feeder cells, Cosmo Bio Co., Ltd., CBA-311) were seeded in a culture vessel. The ultra-low attachment 24-well plate was used as the culture vessel. Mouse fibroblasts were seeded in the culture vessel at a cell density of 2 × 10⁶ cells/cm².

Also when fibroblasts were used, a cell sheet was successfully formed. The diameter was about 1.5 cm. A photograph of the obtained fibroblast sheet is shown in FIG. 8.

The fibroblast sheet had a fibroblast purity of 100%. The fibroblast sheet had a uniform shape. That is, the fibroblast sheet had a substantially uniform thickness over the entire sheet and had an approximately circular shape, when observed with the naked eye. In addition, the fibroblast sheet had sufficient strength and was able to be handled by scooping with a spatula. The fibroblast sheet can be used for applications such as transplantation of cultured skin or connective tissue.

A cell sheet was produced in the same manner as in Example 1, except that human iPS cells (Center for iPS Cell Research and Application, Kyoto University, 253G1 strain) were seeded in a culture vessel. The ultra-low attachment 24-well plate (Corning Inc.) was used as a culture vessel. Human iPS cells were seeded in the culture vessel at a cell density of 2.5 × 10⁶ cells/cm².

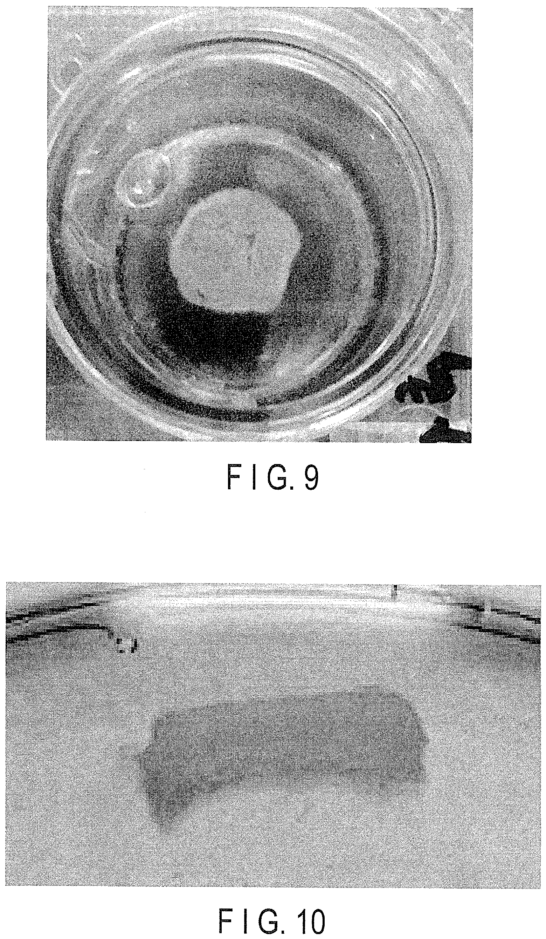

Also, when undifferentiated iPS cells were used, a cell sheet was successfully formed. The diameter was about 0.7 cm. A photograph of the obtained iPS cell sheet is shown in FIG. 9.

The iPS cell sheet had an iPS cell purity of 99%. The iPS cell sheet had a uniform shape. That is, the iPS cell sheet had a substantially uniform thickness over the entire sheet and had an approximately circular shape, when observed with the naked eye. In addition, the iPS cell sheet had sufficient strength and was able to be handled by scooping with a spatula. The iPS cell sheet can be used for various types of tissue differentiation culture.

### [Example 5] Production of quadrangular cell sheet

A cardiomyocyte sheet was produced in the same manner as in Example 1, except that an ultra-low attachment T25 flask (Corning Inc.) was used as the culture vessel. Cardiomyocytes were seeded in the culture vessel at a cell density of 3 × 10⁶ cells/cm².

Also, when a vessel having a quadrangular bottom surface was used, a cardiomyocyte sheet was successfully formed. The size of the cardiomyocyte sheet was about 1.0 × about 2.5 cm. A photograph of the obtained cardiomyocyte sheet is shown in FIG. 10.

The cardiomyocyte sheet had a cardiomyocyte purity of 95%. The cardiomyocyte sheet had a uniform shape. That is, the cardiomyocyte sheet had a substantially uniform thickness over the entire sheet and had a quadrangular shape similar to that of the bottom surface of the vessel, when observed with the naked eye. In addition, the cardiomyocyte sheet had sufficient strength and was able to be handled by scooping with a spatula.

The above results demonstrate that the method of the present invention can freely control the shape of the cell sheet by changing the shape of the bottom surface of the culture vessel.

### [Example 6] Production of human mesenchymal stem cell sheet

A cell sheet was produced in the same manner as in Example 1, except that human mesenchymal stem cells (hMSC LONZA PT-2501) were seeded in a culture vessel. An ultra-low attachment 6-well plate was used as the culture vessel. Human mesenchymal stem cells were seeded in the culture vessel at a cell density of 2 × 10⁶ cells/cm².

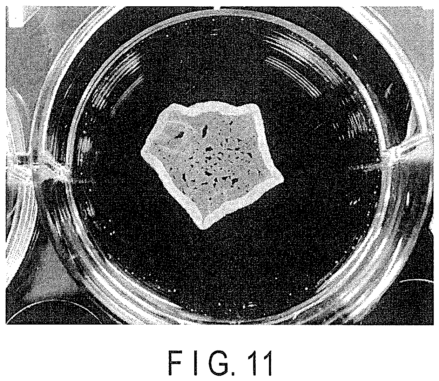

Also, when human mesenchymal stem cells were used, a cell sheet was successfully formed. The maximum length was about 1.8 cm. A photograph of the obtained human mesenchymal stem cell sheet is shown in FIG. 11.

The human mesenchymal stem cell sheet had a cell purity of 100%. The human mesenchymal stem cell sheet had a relatively uniform shape. That is, the human mesenchymal stem cell sheet had a substantially uniform thickness over the entire sheet and had an approximately circular shape, when observed with the naked eye. In addition, the human mesenchymal stem cell sheet had sufficient strength and was able to be handled by scooping with a spatula. The human mesenchymal stem cell sheet can be used for applications such as transplantation to myocardial infarction, lower limb ischemia, or a wound.

## Claims

1. A method for producing a cell sheet, the method comprising subjecting animal cells dispersed in a culture medium to a suspension culture while gathering the animal cells toward the center of the culture medium, to form a cell sheet floating in the culture medium.

2. The method according to claim 1, wherein the suspension culture is performed by applying vibration to the culture medium.

3. The method according to claim 2, wherein the vibration is a vibration in a direction substantially perpendicular to a direction of gravity.

4. The method according to claim 2 or 3, wherein the vibration is a rotational vibration.

5. The method according to any one of claims 1 to 4, wherein the culture medium comprises a thickening agent.

6. The method according to any one of claims 1 to 5, wherein the animal cells are present at a cell density of 0.1 × 10⁶ to 10 × 10⁶ cells/cm² at a start of the suspension culture.

7. The method according to any one of claims 1 to 6, wherein the animal cells comprise iPS cell-derived differentiated cells.

8. The method according to any one of claims 1 to 7, wherein the animal cells comprise iPS cell-derived cardiomyocytes.

9. The method according to any one of claims 1 to 8, wherein the animal cells comprise iPS cell-derived cardiomyocytes at a cardiomyocyte purity of 80 to 100%, preferably 90 to 100%.

10. The method according to any one of claims 1 to 6, wherein the animal cells are mesenchymal stem cells.

11. A cardiomyocyte sheet having a cardiomyocyte purity of 80 to 100%, preferably 85 to 100%, more preferably 90 to 100%, still more preferably 95 to 100%, and most preferably 98 to 100%.

12. The cardiomyocyte sheet according to claim 11, wherein the cardiomyocyte sheet has a uniform shape.

13. The cardiomyocyte sheet according to claim 11 or 12, wherein the cardiomyocyte sheet is free from a cell other than cardiomyocytes.

14. The cardiomyocyte sheet according to any one of claims 11 to 13, wherein the cardiomyocyte sheet has a maximum length of 0.1 to 30 cm, preferably 1 to 10 cm.

15. A kit for producing a cardiomyocyte sheet, the kit comprising:
iPS cell-derived cardiomyocytes;
a culture medium for culturing the cardiomyocytes; and
a suspension culture vessel for containing the culture medium.

16. The kit according to claim 15, wherein the vessel has a coating for inhibiting cell adhesion applied to an inner wall of the vessel.

17. The kit according to claim 15 or 16, wherein the cardiomyocytes have a cardiomyocyte purity of 80 to 100%, preferably 85 to 100%, more preferably 90 to 100%, still more preferably 95 to 100%, and most preferably 98 to 100%.
